# EUROPEAN PATENT SPECIFICATION

(11) **EP 2 131 738 B1**
(45) Date of publication and mention of the grant of the patent: **06.11.2013**
(21) Application number: 08720060.6
(22) Date of filing: 30.03.2008
(51) Int. Cl.: A61B 5/1455

(54) **A METHOD AND APPARATUS FOR ENHANCEMENT AND QUALITY IMPROVEMENT OF ANALYTE MEASUREMENT SIGNALS**
VERFAHREN UND GERÄT ZUR VERSTÄRKUNG UND VERBESSERUNG DER QUALITÄT VON ANALYTEN-MESSSIGNALEN
PROCEDE ET APPAREIL PERMETTANT DE RENFORCER ET D'AMELIORER LA QUALITE DE SIGNAUX DE MESURE D'ANALYTE

(30) Priority: 04.04.2007 US 696715
(43) Date of publication of application: 16.12.2009
(73) Proprietor: Orsense Ltd., 74036 Ness Ziona (IL)
(72) Inventor: FINAROV, Alexander, 76302 Rehovot (IL); KLEINMAN, Yossie, 76608 Rehovot (IL)
(74) Representative: Jennings, Tara Romaine
(86) International application number: PCT/IL2008/000438
(87) International publication number: WO 2008/122973

(56) References cited:
- EP-A- 0 313 238
- WO-A-2007/030379
- US-B1- 6 385 821
- US-B1- 6 400 972

## Description

### TECHNICAL FIELD

The present device is in the field of medical instrumentation and, in particular, non-invasive measurements of physiological parameters of subjects.

### BACKGROUND

In recent years, several techniques have been proposed for non-invasive determination of physiological parameters of patients or objects, such as oxygen saturation, hemoglobin, glucose, bilirubin, cholesterol and others that collectively may be termed *analytes*. Among the methods frequently used are methods that utilize light and especially Red and Near Infrared (RNIR) radiation that is transmitted or reflected from a blood perfused fleshy medium. (In the text of the present disclosure, light is interpreted as electro-magnetic radiation.) Usually, the radiation consists of a plurality of wavelengths selected from a broad radiation spectrum. Each analyte responds differently to different wavelengths. Analyses of absorption, scattering, transmission or reflection of different wavelengths by blood, interstitial fluids, tissue, or blood perfused fleshy medium, will all be henceforth termed radiation-object interaction products, and assist in determination of the desired analyte concentration.

The sources of RNIR radiation and the corresponding detectors are usually embedded in different shapes and configurations in a probe attached to a measurement object, which is typically the subject's finger, earlobe, or other part of the body. Both the probe and the object should be stable and maintain their relative position in the course of the measurement. Minute relative movements between the probe and subject, which occur during the measurement and are so called *motion artifacts*, may distort the measurement results. Complicated algorithms, such as one disclosed in United States Patent No.5,743,262 to Lepper et al., or Masimo, Inc., publication # LAB1035M (www.massimo.com), are applied to reduce the influence of motion artifacts.

EP 313 238 discloses a pulse oximeter comprising a multi-layer finger wrap with an adhesive layer to secure the wrap about the finger.

In order to keep the probe in a stable relation to the object, some of the probes, e.g., disclosed in United States Patents Nos. 6,461,305 and 6,488,633 to Schnall, apply a certain pressure to the object. These probes fall short in preventing relative displacement between the object and the probe. The pressure distribution by itself may be a source of additional measurement errors.

Consequently, it is desirable to have a probe, a method of using the probe for physiological parameter measurement, and an apparatus implementing such method that would be free or substantially reduce the influence of motion artifacts.

### SUMMARY

The present invention provides a solution to the above-described needs in the art, as well as other needs by, in general, providing a probe for the measurement of analytes that operates to reduce the relative motion between the object from which the measurements are being taken and the probe. Several embodiments of the present invention are presented herein and each such embodiment, although it may be a patentable invention in and of itself, is presented as a non-limiting example of the present invention. For instance, in one embodiment of the invention, a sticky material is applied in a variety of manners to either a surface of the probe that is in contact with the object, or to the object itself. In other embodiments, sticky pads or flexible pins are used to help maintain the relative position between the probe and the object.

Another aspect of the present invention is the incorporation of pressure devices into the probe. The pressure devices serve at least two purposes. One purpose is to further restrict relative movement of the object and probe. However, another purpose is to provide for the restriction or even cessation of blood flow. For instance, an occlude-release cycle mode can be created and the analyte measurements can be taken during this cycle or at strategic points in the cycle depending on the analyte being measured.

Further embodiments, aspects and features of the present invention are presented in the detailed description. It will be appreciated that not all of the aspects, features and embodiments presented are necessary elements of the invention and in fact, although various embodiments may be individually patented, the present invention is not limited to any particular set of features and/or aspects.

### BRIEF LIST OF DRAWINGS

The disclosure is provided by means of non-limiting examples only, with reference to the accompanying drawings. The drawings are not necessarily to scale, emphasis instead being placed upon illustrating the principles of the devices and methods.

Figures 1A and 1B are schematic illustrations of some exemplary embodiments of the probe.

Figures 2A - 2D are schematic illustrations of a cross section of one of the exemplary embodiments of the probe.

Figure 3 is a schematic illustration of an additional exemplary embodiment of the probe.

Figure 4 is a schematic illustration of yet another exemplary embodiment of the probe.

Figures 5A - 5D are schematic illustrations of further exemplary embodiments of the probe.

Figure 6 is a schematic illustration of an exemplary method of application of the probe.

Figures 7A and 7B are schematic illustrations of an exemplary method of application of the probe and a pressure application article for measurement of physiological parameter.

Figure 8 is a schematic illustration of an exemplary embodiment of the probe combined with a pressure application article.

Figures 9A and 9B are schematic illustrations of additional exemplary embodiments of the application of a sticky surface for measurement of physiological parameters.

Figure 10 is a schematic illustration of a double sticky wrapping for the measurement of physiological parameter.

### DETAILED DESCRIPTION OF THE EXEMPLARY EMBODIMENTS

The method and apparatus constructed according to the method may be understood with reference to the drawings and the accompanying description, wherein like numerals of reference designate like elements throughout the text of the disclosure. In this regard, directional terminology, such as "top," "bottom," "front," "back," "upper," "lower," etc., is used with reference to the orientation of the Figure(s) being described. Because components of embodiments of the present probe can be positioned in a number of different orientations, the directional terminology is used for purposes of illustration and is in no way limiting.

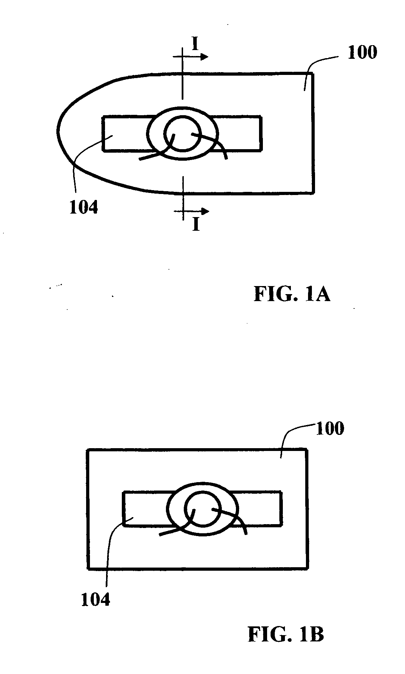

Reference is made to Figures 1A and 1B, which are schematic illustrations of some exemplary embodiments of the probe that may be used in non-invasive patient or object physiological parameters determination. Probe 100 has a body that may be of a thimble-like shape (Figure 1 A) or sleeve-like shape (Figure 1B). Probe 100 may have one or more ribs 104 increasing the stiffness of the structure. Depending on the desired stiffness, ribs 104 may be located on upper and lower halves of probe 100.

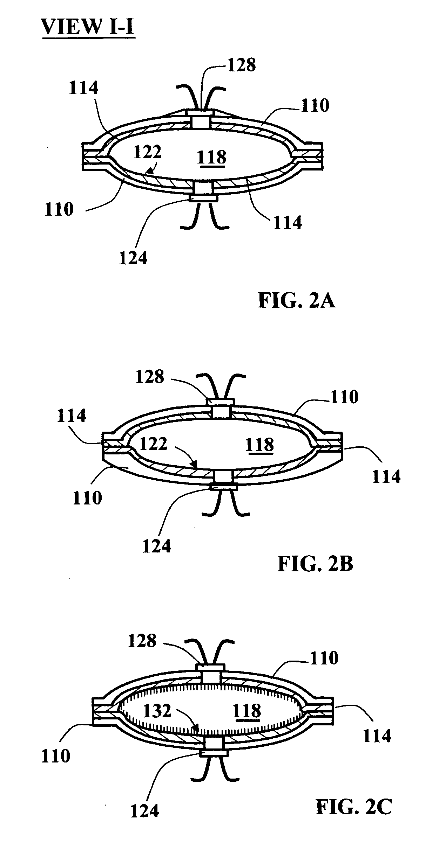

Figures 2A - 2D are schematic illustrations of a cross section of an exemplary embodiment of the probe. The body of probe 100 is a resilient structure consisting of an outer layer 110 and inner layer 114. The body may have a symmetric form (Figure 2A), or an asymmetric form as shown in Figure 2B. Outer layer 110 is stiffer than inner layer 114 and it develops and supports uniform pressure on the object (not shown) when the object is inserted in the opening 118 of probe 100. Surface 122 of inner layer 114 that engages the object when it is inserted in opening 118, possess sticky properties. As will be explained below the sticky properties may be achieved by use of proper materials, material processing, or coating. They should be such that when inserted in the probe the object adheres temporarily to the sticky surface 122 and an effort is required to reposition it or cause relative movement between the object and probe 100. Because of this, the motion artifacts caused by relative movement between the probe and the object are eliminated or substantially reduced and do not affect the measurement process.

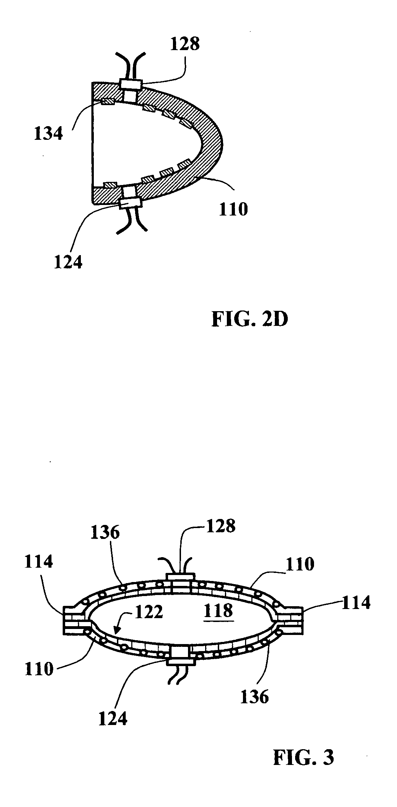

A source of radiation 124 such as an incandescent lamp, LED, Laser or VCSEL, and a suitable radiation detector 128, are built into probe 100. Figure 2 and subsequent figures show a radiation source 124 and detector 128 locations adapted for transmission measurement. It should be clear, however, that their location may be adapted to measure reflection from the object and other radiation-object interaction products. Multiple sources and detectors measuring both the reflection and transmission may be incorporated in probe 100 as well. Typically, the sources produce radiation in a wavelength range between 400 nm and 2500 nm. Usually, at least two wavelengths are used for a measurement.

The outer layer 110 and inner layer 114 may be manufactured from silicone or similar material and joined by application of adhesive, lamination, or any other known method. The mechanical properties of the outer layer 110, however, are preferably different from the mechanical properties of the inner layer 114. For example, the hardness of the inner silicone layer could be in the range of Shorr value of 5 - 10, where the outer layer may have hardness values exceeding Shorr 60 or more. Silicone having hardness value of Shorr 15 and lower has a sticky surface that adheres practically to everything, although the strength of adhesion may be different among different materials and surfaces.

In an alternative embodiment, illustrated in Figure 2C, inner layer 114 may be manufactured from silicone having a large number of protruding columns or needles 132 of few tenths of micron size. The height and elasticity of silicone columns 132 should be such as to enable fixation on the object without damaging it. In one of the embodiments, illustrated in Figure 2D, inner layer may be made of sections 134 of sticky material. The amount of sections 134 and their surface should be sufficient to hold the object and restrict a relative movement between the object and the probe and in particular between object and radiation source 124 and detector 128. A sleeve-like probe may be implemented in a similar way.

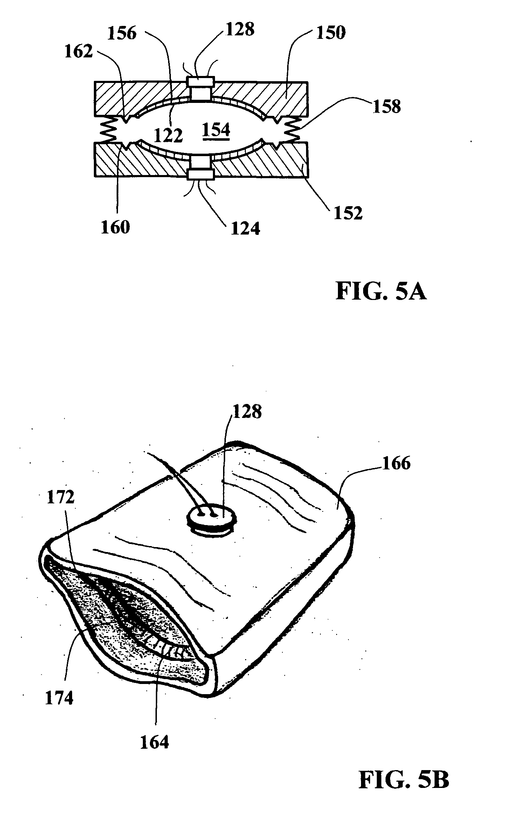

The optical signal measured by the detector 128 is a weak one and typically is affected by ambient illumination. Lightproof silicone or similar material should be used for at least one of the layers 110 or 114 of probe 100. Opening 118 that receives the object may be shielded from ambient illumination by adding light proof baffles or making a ring of porous material 164 (Figure 5B) at the end of probe 100. Silicone columns 132 may be implemented to have a height that would allow shielding of the opening 118 that receives the object, from ambient light. Columns 132 allow free passage of air and reduce or eliminate object sweating that occurs when it is inserted into the probe 100. Alternatively, lightproof air permeable material may be used as a layer in the manufacturing of probe 100. A large selection of porous synthetic material, such as acrylic materials, Dacron, porous polyethylene, Proplast II and other similar materials may be suitable for such task.

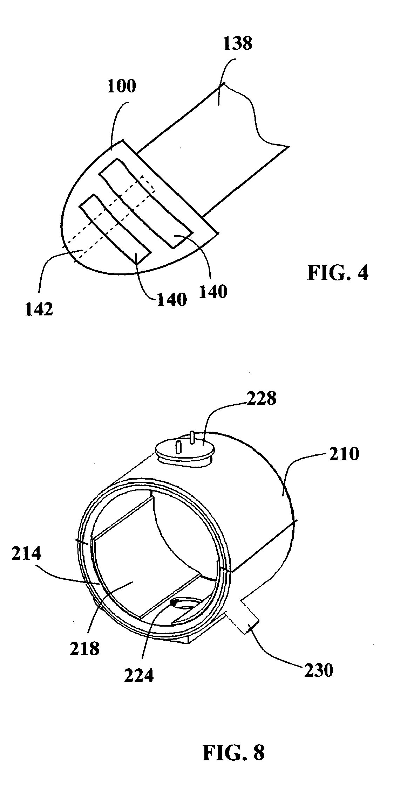

To sit firm on an object, probe 100 should develop certain pressure, generally uniformly distributed over the surface of object. Figure 3 is a schematic illustration of an additional exemplary embodiment of the present probe. Depending on the pressure required, probe 100 may be armored by spring steel strips 136 and linear or spiral springs. Steel strips 136 may be embedded or molded together with the outer layer 110, or the inner layer 114, or located between the layers. As shown in Figure 4, steel or plastic strips 140 or 142 may be external to probe 100. Other numerals in Figure 4 mark the object 138 inserted in probe 100 and circumferential spring strip 140 or longitudinal springs 142. The internal or external armored elements are effective in generating uniform pressure and enhancing the movement restricting effect of the sticky surface. The size and stiffness of the armored elements may be selected such as to ensure that the pressure developed by them does not substantially affect the measurement results.

Figures 5A through 5D are schematic illustrations of further exemplary embodiments of the present invention. Figure 5A illustrates a probe 148 that includes an upper 150 and a lower 152 halves and an opening 154. Linings 156 made of sticky material or strips of sticky material, or at least having a sticky surface 122 are attached to the inner surfaces of the upper and lower halves. Springs 158 ensure the desired pressure and groves 160 with protrusions 162 ensure that the opening 154 is light proof when an object is inserted into it.

Figure 5B is a schematic illustration of probe 166 fabricated as two symmetric or asymmetric halves 168 and 170 of resilient light proof material. Probe 166 has an opening 172 that receives the measurement object (not shown). Before each measurement a sticky spray, such as Repositionable Adhesive Spray 75, commercially available from 3M, Saint Paul MN, USA is applied such as to cover the inner surface 174 of opening 172. Upon completion of measurement, the spray may be removed by alcohol.

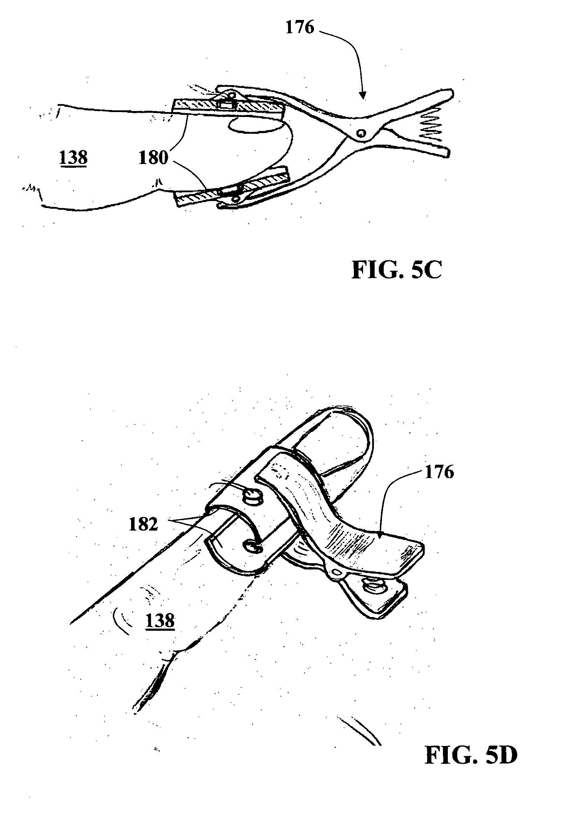

Figures 5C and 5D are schematic illustrations of additional clip-like probe embodiments. Clip-like probes 176 and 178 respectively have the surfaces 180 and 182 being in contact with the object 138, such as finger or earlobe, and are made of sticky material, or as disclosed above, coated on demand by sticky coating.

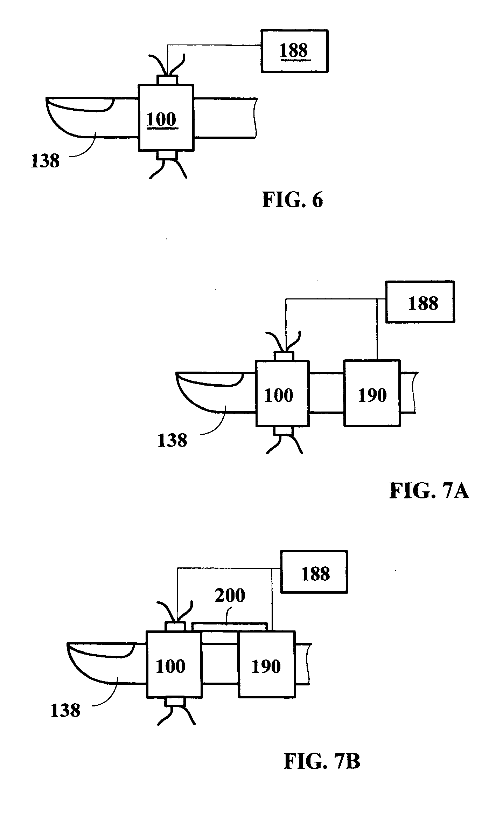

Figure 6 is a schematic illustration of an exemplary method of application of the present probe. An object 138 such as a finger is inserted into a sleeve shaped probe 100. Both the radiation source 124 and detector 128 are connected to a controller 188 that operates them, receives measurement results, processes the results according to a certain algorithm, interprets the results of processing in terms of analyte concentration and displays the results or sends them into a displaying device. The measured analyte is a tissue and blood analyte and may be the concentration of one of hemoglobin, hematocrit, glucose, bilirubin, oxygen saturation, and other blood and tissue analytes. Upon completion of the measurements for one subject, the inner surface 114 of the probe may be cleaned by alcohol or similar fluid, where dirt and sweat left by the previous patient are removed, and the surface stickiness restored.

The measurement scheme of Figure 6 may be sufficient for certain blood and tissue analyte determination applications for example, such as oximetry, or lower accuracy hemoglobin and the measurements of other analytes. As noted above, the optical signal measured by the detector is a weak one and typically has a poor signal to noise ratio. In order to improve the signal to noise ratio of the measured signal and make the measurement more reliable and suitable for measurements of glucose and hemoglobin, methods of blood and interstitial fluids flow intensification are used. These methods include change of temperature at the measurement point, application of pressure, including occlusion and cessation of blood flow, application of materials causing local stimulation and others.

United States Patents Nos. 6,213,972, 6,400,977, 6,711,424 and 6,804,002 all presently held by the assignee of the present application, disclose different types of finger holders that in addition to regular operation, operate in pressure-release (occlusion-release) mode, under which certain pressure is applied to the finger. The pressure, which is released after a predetermined time, may include an over systolic pressure that occludes vessels and ceases the blood flow at the measurement location. The measurements may be taken through the entire pressure-release cycle, or at predetermined time intervals. As used herein the pressure-release cycle may include an occlusion-release cycle.

Figure 7A is a schematic illustration of an exemplary method of application of the present probe and a pressure application article for measurement of physiological parameter. Object 138 is inserted into a pressure-developing article 190 such as a pneumatic cuff and into a probe 100. The controller 188 operates the radiation source, detector and the pressure sequence applied by pressure developing article 190 that enhances fluids flow and improves signal to noise ratio. Pressure developing article 190 develops a range of pressures including over-systolic pressure that occludes blood-conducting vessels and ceases blood flow. Controller 188 receives the measurement results, processes the results according to certain a algorithm, and interprets the results of processing in terms of analyte concentration. Controller 188 may include a display for visual representation of the measurement results. The display provides an instant feedback to the caregiver or person operating the apparatus. The measured analyte concentration may be one of hemoglobin, hematocrit, glucose, bilirubin, oxygen saturation, cholesterol and albumin as well as others blood or tissue analytes.

A strip 200 made of rigid material (Figure 7B) connects between probe 100 and pressure developing article 190 and forms a unit similar to a probe. Strip 200 reduces or eliminates motion artifacts that may be produced by occasional relative movement of the probe and article caused by incidental movement of finger 138 phalanx.

In a further embodiment illustrated in Figure 8, the pressure-developing article is incorporated into a sleeve like body of probe 210. The particular pressure-developing article is a pneumatic cuff 214 with the surface 218 of the cuff engaging the object, and is similarly a sticky surface, produced by any one of the discussed above methods. Other than pneumatic cuffs, pressure applications devices may be also used. Two mechanically locked halves of article 210 allow easy insertion of the object. Probe 210 includes one or more radiation sources 224 and at least one detector 228 arranged to measure the radiation-object interaction products. Pipe socket 230 facilitates connection to a source of compressed air for pneumatic cuff 214. Probe 210, by means of cuff 214, may operate in a pressure-release or occlusion-release cycle and the measurements may be taken through the entire pressure-release cycle, or at predetermined time intervals.

Measurement of certain analytes has a lower accuracy, and may not require application of pressure. In such cases, probe 210 will operate in a conventional mode with the sticky surface reducing or eliminating the influence of motion artifacts.

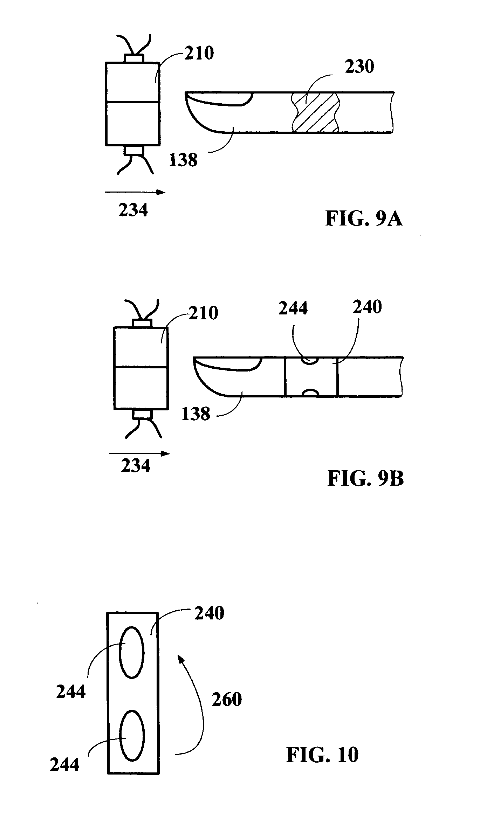

In another exemplary embodiment of the method of application of a sticky surface for measurement of physiological parameter illustrated in Figure 9A, Repositionable Adhesive Spray 75 may be sprayed around the phalanx of the finger 138 to form a sticky coating 230 on the finger. Probe 210, 100 or any other similar probe that engages the measurement object may receive the finger and engage the sticky surface. Sticky coating 230 ensures absence of movement during the measurement of a physiological parameter, which may be a tissue or blood analyte, between the probe and the object (finger 138). Spray 75 is easy to remove by cleaning the finger by alcohol or warm water.

Figure 9B illustrates an additional exemplary embodiment of the method of application of a sticky surface for measurement of physiological parameter. In this embodiment a double sticky transparent tape, serving as a wrapping wrapped around finger 138 may also be applied for measurement of physiological parameter. In another embodiment an opaque tape 240 having windows 244 matching the location of the radiation source and detector may be wrapped around the finger or located on an earlobe. Double sticky tape 240 greatly reduces any movement during the measurement of a physiological parameter between the probe and the object.

Figure 10 is a schematic illustration of a double sticky wrapping for measurement of physiological parameters. Wrapping 240 has windows 244 located such that when wrapped as shown by arrow 260 around finger 138 windows 244 match the location of the radiation source and detector. Wrappings 240 may be supplied as a tape with creasing allowing for convenient detachment of a particular section of the tape, or as individual wrappings of a number of sizes matching finger sizes of different objects.

The probe disclosed and associated with it physiological parameters determination method significantly reduces and in some cases eliminates motion artifacts' influence, improves measurement reliability, and reduces the number of faulty measurements, consequently resulting in a higher-accuracy measurement with increased comfort to the subject.

Although specific embodiments have been illustrated and described herein, it will be appreciated by those of ordinary skill in the art that a variety of alternate and/or equivalent implementations may be substituted for the specific embodiments shown and described without departing from the scope of the present probe and the disclosed method. This application is intended to cover any adaptations or variations of the specific embodiments discussed herein. Therefore, it is intended that these probe and method be limited only by the claims and the equivalents thereof.

## Claims

1. A probe (100) for non-invasive determination of physiological properties of an object, the probe (100) comprising:
an opening (118) for receiving an object;
a body being a resilient structure consisting of:
an inner layer (114) having a surface (122) possessing sticky properties and adapted to engage the object when the object is inserted in the opening;
an outer layer (110) being stiffer than the inner layer (114) and adapted to develop and support uniform pressure on the object when the object is inserted in the opening (118);
the probe further comprising:
a source of radiation (124) and a detector (128) the detector being built into the probe.

2. The probe (100) according to claim 1, wherein said surface (122) possessing sticky properties is one of a silicone layer having a hardness of less than 15 Shorr surface, a plurality of micron size columns, or a temporarily sprayed coating.

3. The probe (100) according to claim 1, wherein said surface (122) possessing sticky properties is a cleanable surface.

4. The probe (100) according to claim 1, wherein said source of radiation (124) is a source of red and infrared radiation.

5. The probe (100) according to claim 1, wherein the probe (110) is armored by one of external or internal armor elements generating uniform pressure and enhancing a movement restriction effect of surface (122) possessing sticky properties.

6. The probe (100) according to claim 5, wherein the armored elements of the probe (100) are steel springs (136) embedded or molded together with one of a group of layers consisting of the outer layer (110), the inner layer (114), or located between the outer and inner layers.

7. The probe (100) according to claim 1, wherein said body is a lightproof thimble-like or sleeve-like body.

8. The probe according to claim 1, wherein the probe (100) further comprises a pressure development article (190) configured to operate in a pressure sequence that enhances fluids flow and improves signal to noise ratio.

9. The probe according to claim 8, wherein the pressure sequence enhances fluids flow and improves signal to noise ratio is operatively configured to operate in occlusion-release mode, and wherein said occlusion-release mode develops pressure higher than systolic pressure.

10. The probe according to any preceding claim, wherein said physiological parameter is concentration of one of hemoglobin, hematocrit, glucose, bilirubin, oxygen saturation, cho lesterol and albumin.

11. An apparatus comprising a probe according to any one of claims 1 - 10.

12. A method of reducing motion artifacts in a non-invasive analyte concentration measurement, said method comprising:
a) applying to a measurement object a probe according to claim 1 with a surface engaging said object being a sticky surface; and
b) operating said probe to perform the measurement of said analyte of interest in occlude-release mode, said method **characterised in that** said sticky surface is adapted to reduce said motion artifacts by restricting with the help of said sticky surface said object-probe relative movements.

13. The method according to claim 12, wherein the operation of said probe in said occlude-release mode develops pressure that exceeds systolic pressure and temporarily ceases blood flow in said object.

14. The method according to claim 12, wherein operation of said probe includes operation of a source of red and infrared radiation that is built into said probe.

15. The method according to claim 12, wherein said analyte of interest is concentration of one of hemoglobin, hematocrit, glucose, bilirubin, oxygen saturation, cholesterol and albumin.

## Patentansprüche

1. Sonde (100) zur nichtinvasiven Bestimmung von physiologischen Eigenschaften eines Objekts, wobei die Sonde (100) Folgendes umfasst:
eine Öffnung (118) zur Aufnahme eines Objekts;
einen Körper, der eine belastbare Struktur aufweist, die aus Folgenden besteht:
einer inneren Schicht (114), die eine Oberfläche (122) aufweist, die Klebeeigenschaften besitzt und geeignet ist, um das Objekt zu erfassen, wenn das Objekt in die Öffnung eingeführt wird;
einer äußeren Schicht (110), die steifer ist als die innere Schicht (114) und geeignet ist, gleichmäßigen Druck auf das Objekt zu entwickeln und zu halten, wenn das Objekt in die Öffnung (118) eingeführt wird;
wobei die Sonde weiter Folgendes umfasst:
eine Strahlungsquelle (124) und einen Detektor (128), wobei der Detektor in die Sonde eingebaut ist.

2. Sonde (100) nach Anspruch 1, worin die genannte Oberfläche (122), die Klebeeigenschaften besitzt, eine einer Silikonschicht ist, die eine Oberfläche mit einer Härte von weniger als 15 Shore, eine Vielzahl an Säulen in Mikrongröße oder eine temporär aufgespritzte Beschichtung aufweist.

3. Sonde (100) nach Anspruch 1, worin die genannte Oberfläche (122), die Klebeeigenschaften besitzt, eine reinigungsfähige Oberfläche ist.

4. Sonde (100) nach Anspruch 1, worin die genannte Strahlungsquelle (124) eine Quelle für Rot- und Infrarotstrahlung ist.

5. Sonde (100) nach Anspruch 1, worin die Sonde (110) durch eines von externen oder internen Verstärkungselementen verstärkt ist, die gleichmäßigen Druck erzeugen und einen Bewegungseinschränkungseffekt der Oberfläche (122), die Klebeeigenschaften besitzt, erhöhen.

6. Sonde (100) nach Anspruch 5, worin die Verstärkungselemente der Sonde (100) Stahlfedern (136) sind, die zusammen mit einer von einer Gruppe von Schichten eingebettet oder verbunden sind, die aus der äußeren Schicht (110), der inneren Schicht (114) besteht, oder die sich zwischen der äußeren und inneren Schicht befinden.

7. Sonde (100) nach Anspruch 1, worin der genannte Körper ein lichtundurchlässiger fingerhutähnlicher oder schlauchartiger Körper ist.

8. Sonde nach Anspruch 1, worin die Sonde (100) weiter einen Druckentwicklungsartikel (190) umfasst, der konfiguriert ist, um in einer Drucksequenz zu betreiben, die den Flüssigkeitsfluss erhöht und das Signal-Rausch-Verhältnis verbessert.

9. Sonde nach Anspruch 8, worin die Drucksequenz, die den Flüssigkeitsfluss erhöht und das Signal-Rausch-Verhältnis verbessert, funktionsfähig konfiguriert ist, um im Okklusions-Freisetzungs-Modus zu betreiben, und worin der genannte Okklusions-Freisetzungs-Modus einen höheren Druck als den systolischen Druck entwickelt.

10. Sonde nach einem vorstehenden Anspruch, worin der genannte physiologische Parameter die Konzentration von einem der Folgenden ist: Hämoglobin, Hämatokrit, Glycose, Bilirubin, Sauerstoffsättigung, Cholesterin und Albumin.

11. Vorrichtung, die eine Sonde nach einem der Ansprüche 1-10 umfasst.

12. Verfahren zur Verringerung von Bewegungsartefakten bei einer nichtinvasiven Messung einer Analytenkonzentration, wobei das genannte Verfahren Folgendes umfasst:
a) Anwenden auf ein Messobjekt einer Sonde nach Anspruch 1 mit einer Oberfläche, die eine klebrige Oberfläche ist, die das genannte Objekt erfasst; und
b) Betreiben der genannten Sonde, um die Messung des genannten interessierenden Analyten im Okklusions-Freisetzungs-Modus auszuführen, wobei das genannte Verfahren **dadurch gekennzeichnet ist, dass** die genannte klebrige Oberfläche geeignet ist, um die genannten Bewegungsartefakten durch Einschränkung der genannten relativen Objekt-Sonde-Bewegungen mithilfe der genannten klebrigen Oberfläche zu verringern.

13. Verfahren nach Anspruch 12, worin das Betreiben der genannten Sonde im genannten Okklusions-Freisetzungs-Modus Druck entwickelt, der den systolischen Druck übersteigt und den Blutfluss im genannten Objekt temporär einstellt.

14. Verfahren nach Anspruch 12, worin das Betreiben der genannten Sonde das Betreiben einer Quelle für Rot- und Infrarotstrahlung einschließt, die in die genannte Sonde eingebaut ist.

15. Verfahren nach Anspruch 12, worin der genannte interessierende Analyt die Konzentration von einem der Folgenden ist: Hämoglobin, Hämatokrit, Glycose, Bilirubin, Sauerstoffsättigung, Cholesterin und Albumin.

## Revendications

1. Sonde (100) pour la détermination non invasive de propriétés physiologiques d'un objet, la sonde (100) comprenant :
une ouverture (118) pour recevoir un objet ;
un corps étant une structure résiliente consistant en :
une couche interne (114) ayant une surface (122) possédant des propriétés collantes et adaptée pour mettre en prise l'objet quand l'objet est inséré dans l'ouverture ;
une couche externe (110) plus rigide que la couche interne (114) et adaptée pour développer et supporter une pression uniforme sur l'objet quand l'objet est inséré dans l'ouverture (118) ;
la sonde comprenant en outre :
une source de rayonnement (124) et un détecteur (128), le détecteur étant incorporé à la sonde.

2. Sonde (100) selon la revendication 1, dans laquelle ladite surface (122) possédant des propriétés collantes est l'une d'une couche de silicone ayant une surface d'une dureté de moins de 15 Shore, une pluralité de colonnes de taille micronique, ou un revêtement vaporisé temporairement.

3. Sonde (100) selon la revendication 1, dans laquelle ladite surface (122) possédant des propriétés collantes est une surface nettoyable.

4. Sonde (100) selon la revendication 1, dans laquelle ladite source de rayonnement (124) est une source de rayonnement rouge et infrarouge.

5. Sonde (100) selon la revendication 1, la sonde (110) étant blindée par l'un d'éléments de blindage externe ou interne générant une pression uniforme et rehaussant un effet de restriction de mouvement de la surface (122) possédant des propriétés collantes.

6. Sonde (100) selon la revendication 5, dans laquelle les éléments de blindage de la sonde (100) sont des ressorts en acier (136) noyés ou moulés avec l'une d'un groupe de couches consistant en la couche externe (110), la couche interne (114) ou situés entre les couches externe et interne.

7. Sonde (100) selon la revendication 1, dans laquelle ledit corps est un corps en forme de dé à coudre ou en forme de manchon étanche à la lumière.

8. Sonde selon la revendication 1, dans laquelle la sonde (100) comprend en outre un article de développement de pression (190) configuré pour fonctionner dans une séquence de pression qui rehausse l'écoulement de fluides et améliore le rapport signal/bruit.

9. Sonde selon la revendication 8, dans laquelle la séquence de pression rehaussant l'écoulement de fluides et améliorant le rapport signal/bruit est configurée opérationnellement pour fonctionner en mode occlusion-libération, et dans laquelle ledit mode occlusion-libération développe une pression supérieure à la pression systolique.

10. Sonde selon l'une quelconque des revendications précédentes, dans laquelle ledit paramètre physiologique est la concentration de l'un de l'hémoglobine, l'hématocrite, le glucose, la bilirubine, la saturation en oxygène, le cholestérol et l'albumine.

11. Appareil comprenant une sonde selon l'une quelconque des revendications 1 à 10.

12. Procédé de réduction des artefacts de mouvement dans une mesure non invasive de concentration d'analyte, ledit procédé comprenant :
a) l'application sur un objet de mesure d'une sonde selon la revendication 1, une surface mettant en prise ledit objet étant une surface collante ; et
b) l'actionnement de ladite sonde pour effectuer la mesure dudit analyte d'intérêt en mode occlusion-libération, ledit procédé étant **caractérisé en ce que** ladite surface collante est adaptée pour réduire lesdits artefacts de mouvement en restreignant à l'aide de ladite surface collante lesdits mouvements relatifs objet-sonde.

13. Procédé selon la revendication 12, dans lequel le fonctionnement de ladite sonde dans ledit mode occlusion-libération développe une pression qui dépasse la pression systolique et arrête temporairement le flux de sang dans ledit objet.

14. Procédé selon la revendication 12, dans lequel le fonctionnement de ladite sonde comporte le fonctionnement d'une source de rayonnement rouge et infrarouge qui est incorporée à la sonde.

15. Procédé selon la revendication 12, dans lequel ledit analyte d'intérêt est la concentration de l'un de l'hémoglobine, l'hématocrite, le glucose, la bilirubine, la saturation en oxygène, le cholestérol et l'albumine.
